# EUROPÄISCHE PATENTSCHRIFT

(11) **EP 0 392 498 B1**
(45) Veröffentlichungstag und Bekanntmachung des Hinweises auf die Patenterteilung: **30.06.1993**
(21) Anmeldenummer: 90106938.5
(22) Anmeldetag: 11.04.1990
(51) Int. Cl.: A61M 16/04, A61M 25/02

(54) **Halterung für einen Endotrachealtubus**
Endotracheal tube holder
Support de tube endotrachéal

(30) Priorität: 14.04.1989 DE 3912376
(43) Veröffentlichungstag der Anmeldung: 17.10.1990
(73) Patentinhaber: ACONVEST AG, CH-4001 Basel (CH)
(72) Erfinder: Piwonka, Peter, D-8000 München 71 (DE)
(74) Vertreter: Vogeser, Werner, Dipl.-Ing.

(56) Entgegenhaltungen:
- DE-U- 8 811 131
- GB-A- 2 039 744
- US-A- 4 744 358
- US-A- 4 774 946

## Beschreibung

Die Erfindung betrifft eine Halterung für einen Endotrachealtubus, die an Tubus-Halteband mit einer im Verbindungsbereich mit dem Tubus mittels einer Abziehfolie abgedeckte Klebstoffschicht aufweist.

Eine derartige Halterung ist aus dem DE-GM 88 11 131 bekannt. Weiterhin ist es bekannt, die Befestigung mittels eines elastischen Bandes vorzunehmen, das im Nackenbereich eines Patienten gebunden wird.

Eine weitere Halterung ist aus der US-A-4,744,358 bekannt, bei der ein Halteband nicht unmittelbar an einem Tubus befestigbar ist, sondern an einem Haltegerät komplizierten Aufbaus, das zunächst am Patienten positioniert werden muß. Anschließend kann ein Tubus mittels Laschen in dem Haltegerät befestigt werden.

Die Verwendung eines Endotrachealtubus ist bei der Durchführung von Operationen mit Anästhesie unerläßlich. Eine sichere und genaue Positionierung des Tubus ist inbesondere wegen der Verletzungsmöglichkeit der Epithelhärchen notwendig. Diese Anforderungen erfüllt grundsätzlich die Halterung nach dem DE-GM 88 11 131, jedoch ist es bei dieser erforderlich, die Klebebandstücke, an denen das Halteband lösbar befestigbar ist, mittels Klebstoff auf der Haut zu befestigen.

Gegen eine Befestigung mittels Klebstoff bestehen insbesondere wegen möglicher Hautallergien Bedenken.

Der Erfindung liegt die Aufgabe zugrunde, eine Halterung zu schaffen, die eine genaue und sichere Positionierung eines Endotrachealtubus ermöglicht, ohne daß eine Befestigung auf der Hautoberfläche erforderlich ist.

Gelöst wird diese Aufgabe gemäß der Erfindung durch die im Anspruch 1 angegebenen Merkmale.

Durch die im Prinzip helmartige Ausbildung der Halterung kann auf die Verwendung von Klebstoff zur Befestigung des Haltebands verzichtet werden. Die elastische Ausbildung des Stirnbandes verleiht der Halterung eine ausreichende Anpassungsfähigkeit. Durch die lösbare Verbindung des Haltebandes mit dem Zugband kann eine sichere und genaue Positionierung des Tubus erreicht werden.

Durch die vorgeschlagene Ausbildung wird erreicht, daß die Befestigung eines Tubus für einen Patienten wenig hinderlich ist, daß der Tubus leicht auswechselbar ist, daß eine sichere Befestigung garantiert wird, daß zumindest der kritische Teil der Halterung leicht sterilisierbar ist, daß die Halterung insgesamt einfach handhabbar ist, daß die Halterung leicht nachstellbar ist, und daß die Halterung eine einfache und damit billige Konstruktion hat.

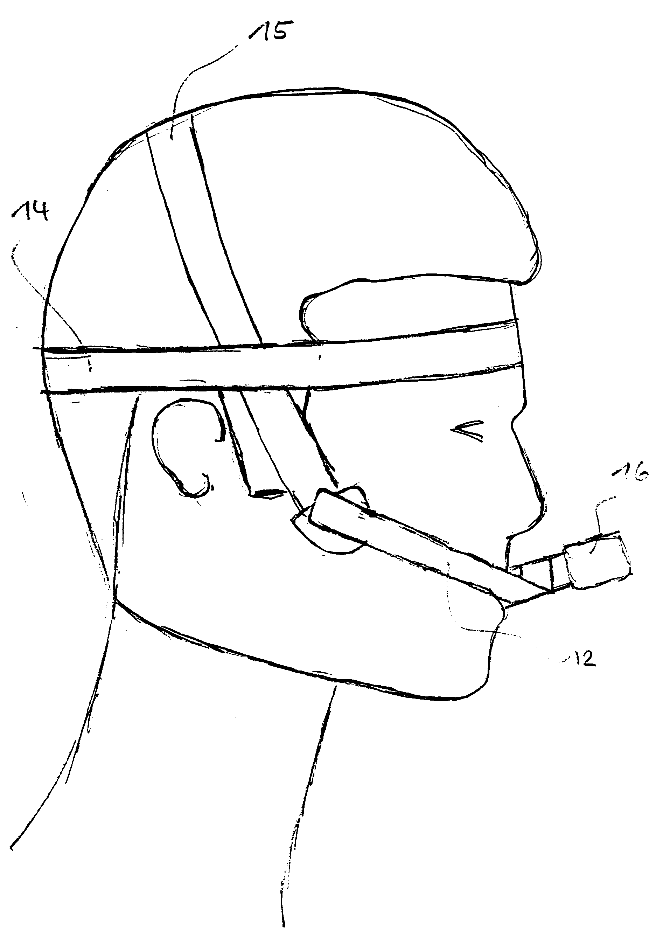

Die Erfindung wird nachstehend anhand der beiliegenden Zeichnung erläutert, aus der die Lage der Halterung am Kopf eines Patienten hervorgeht.

Die Halterung besteht aus einem Halteband 12, das um einen Endotrachealtubus gewickelt ist. In diesem Bereich kann das Halteband 12 mit einer Klebstoffschicht versehen sein, die vor Gebrauch durch eine Abziehfolie abgedeckt ist. Die Enden des Haltebands 12 sind mit einem über den Kopf des Patienten verlaufenden unelastischen Zugband 15 lösbar verbunden, und zwar zweckmäßigerweise mittels eines Klettverschlusses. Hierzu kann das Zugband 15 an den Enden durch einstückig oder gesondert ausgebildete Erweiterungen verbreitert sein.

Das Zugband 15 ist mit einem elastischen Stirnband 14 fest verbunden, so daß das Zugband 15 und das Stirnband 14 eine helmartige Halterung ergeben, die den Patienten wenig behindert und eine ausreichende Anpaßbarkeit hat.

## Patentansprüche

1. Halterung für einen Endotrachealatubus (16) bestehend aus wenigstens einem elastischen Stirnband (14), wenigstens einem mit diesem fest verbundenen und über den Kopf eines Patienten laufenden, unelastischen Zugband (15), dessen Enden als Verbreiterungen ausgebildet und an dessen Enden die Enden eines Tubus-Haltebandes (12) mittels Klettverschluß lösbar befestigbar sind, das im Verbindungsbereich mit dem Tubus (16) eine mittels einer Abziehfolie abgedeckte Klebstoffschicht aufweist.

## Claims

1. Retainer for an endotracheal tube (16), consisting of at least one elastic forehead tape (14), at least one inelastic tension tape (15) which is firmly connected to the latter, which runs over the head of a patient, whose ends are designed as widenings and to whose ends the ends of a retaining tape (12) can be releasibly fastened by means of a hook-and-loop closure which is provided with an adhesive layer which is covered by means of a pull-off film, in the region of connection to the tube (16).

## Revendications

1. Dispositif de maintien de tube endotrachéal (16), constitué par au moins une bande frontale élastique (14), au moins une bande inélastique de traction (15) raccordée rigidement à la bande frontale (14), s'étendant audessus de la tête d'un patient, les extrémités de laquelle sont réalisées sous la forme de parties élargies et aux extrémités de laquelle peuvent être fixées de façon amovible au moyen d'une fermeture à accrochage les extrémités d'une bande de maintien (12), qui a une couche d'adhésif, recouverte au moyen d'une feuille arrachable, dans la zone de jonction avec le tube (16).
